# FASCICULE DE BREVET EUROPEEN

(11) **EP 3 426 215 B1**
(45) Date de publication et mention de la délivrance du brevet: **18.12.2019**
(21) Numéro de dépôt: 16714483.1
(22) Date de dépôt: 08.03.2016
(51) Int. Cl.: A61F 15/00, B26D 1/00, B65H 35/00

(54) **DISTRIBUTEUR POUR ROULEAU DE BANDE DECHIRABLE**
ROLLSPENDER FÜR REISSBARES BAND
TEARABLE TAPE ROLL DISPENSER

(43) Date de publication de la demande: 16.01.2019
(73) Titulaire: Urgo Recherche Innovation et Développement, 21300 Chenove (FR)
(72) Inventeur: LEFEBVRE, Lionel, 21560 Bressey sur Tille (FR)
(74) Mandataire: Plasseraud IP
(86) Numéro de dépôt international: PCT/FR2016/050528
(87) Numéro de publication internationale: WO 2017/153639

(56) Documents cités:
- FR-A- 1 464 897
- GB-A- 388 617
- KR-A- 20090 092 516
- US-A- 2 681 185
- US-A1- 2009 301 660

## Description

L'invention se rapporte à un distributeur pour rouleau de bande déchirable.

En particulier, l'invention se rapporte à un distributeur pour un rouleau de bande déchirable comportant une bande déchirable dont un bout de bande déchirable peut être séparé du rouleau de bande déchirable par une action exercée à la main par un utilisateur. Dans le rouleau de bande déchirable, la bande déchirable est enroulée sur elle-même autour d'un axe central de telle manière que le rouleau de bande déchirable présente un trou selon l'axe central et la bande déchirable présente une extrémité libre. Le trou peut être délimité par un noyau cylindrique sur lequel la bande déchirable est enroulée ou directement par une première spire de bande déchirable au voisinage d'une extrémité opposée à l'extrémité libre.

L'invention s'applique notamment à des rouleaux de bande déchirable utilisés dans le domaine médical ou dans le milieu sportif pour comprimer, immobiliser ou protéger une partie du corps d'une personne. Une bande de ce type est notamment décrite dans le document EP-A-2 058 424. La bande déchirable est réalisée, notamment en termes de structure et de matériau, de manière à présenter un certain nombre de propriétés relatives notamment à son confort, sa résistance, sa respirabilité, sa perméabilité, son maintien et autres.

Pour séparer le bout de bande déchirable du rouleau de bande déchirable, l'utilisateur saisit deux portions de bande déchirable agencées de part et d'autre d'une zone dans laquelle il souhaite obtenir la séparation, et exerce un effort comprenant au moins une composante de cisaillement dans la zone.

Une telle séparation du bout de bande déchirable intervient dans une zone sans qu'il soit possible d'en maîtriser un emplacement précis. La séparation qui en résulte n'est généralement pas nette et le bout de bande déchirable peut présenter une extrémité présentant des irrégularités. L'utilisateur peut alors être amené à séparer successivement plusieurs bouts de bande déchirable jusqu'à ce qu'il juge que les irrégularités sont admissibles. Une certaine quantité de bande déchirable est alors gaspillée. De plus, lorsque le bout de bande déchirable est mis en place, les irrégularités de l'extrémité du bout de bande déchirable peuvent conduire à la formation des plis ou des bourrelets générant une sensation d'inconfort voire des blessures. Les irrégularités de l'extrémité du bout de bande déchirable peuvent également constituer des prises à partir desquels un retrait du bout de bande déchirable peut être amorcé de manière inopinée.

En outre, la séparation d'un bout de bande déchirable du rouleau de bande déchirable nécessite une manipulation complexe ne permettant pas, à l'utilisateur lui-même, de positionner convenablement le bout de bande déchirable sur une partie de son corps.

FR 1 464 897 A illustre également un mode de réalisation de l'état de la technique.

L'invention vise à pallier les problèmes évoqués ci-dessus.

A cet effet, l'invention propose un distributeur comprenant un dévidoir comportant :
- un corps comprenant un support s'étendant selon un axe de pivotement et destiné à être placé dans le trou du rouleau de bande déchirable de manière à permettre un pivotement du rouleau de bande déchirable selon l'axe de pivotement,
- une partie de distribution destinée à recevoir une partie de bande déchirable comportant l'extrémité libre, la partie de distribution comprenant un dispositif de séparation écarté du support et adapté pour séparer du rouleau de bande déchirable un bout de bande déchirable, la partie de distribution comprenant une paroi qui s'étend autour d'un axe de défilement depuis le corps, la paroi de la partie de distribution comportant deux portions d'actionnement en regard l'une de l'autre, dans lequel le dispositif de séparation comprend deux organes d'actionnement actionnables par l'utilisateur, les organes d'actionnement portant respectivement deux bords de séparation en regard l'un de l'autre, les organes d'actionnement étant déplaçables l'un par rapport à l'autre entre une position inactive dans laquelle les bords de séparation sont écartés l'un de l'autre de manière à pouvoir faire défiler la bande déchirable entre lesdits bords de séparation, et une position active dans laquelle les bords de séparation sont rapprochés l'un de l'autre de manière à pouvoir venir en contact avec la bande déchirable,
   le distributeur étant caractérisé en ce que au moins l'un des organes d'actionnement est une languette d'actionnement ménagée sur l'une des portions d'actionnement, la languette d'actionnement étant partiellement séparée d'une partie restante de ladite portion d'actionnement par une découpe et s'étendant depuis une base reliée à ladite partie restante, la languette d'actionnement étant déplaçable entre les positions active et inactive par déformation élastique au niveau de la base.

Ainsi, le distributeur selon l'invention permet d'obtenir une séparation en un emplacement précis qui est défini par l'emplacement auquel les bords de séparation viennent en contact avec la bande déchirable, de part et d'autre de celle-ci. Les bords de séparation assurent une séparation nette du bout de bande déchirable. Le bout de bande déchirable est alors dépourvu d'irrégularité à ses extrémités, ce qui permet de limiter les risques de gaspillage ainsi que les risques d'apparition de plis, bourrelets ou prises et les inconvénients qu'ils présentent.

La manipulation du rouleau de bande déchirable pour en séparer un bout de bande déchirable peut être simplifiée. En particulier, le dévidoir peut être portable à la main par l'utilisateur et les organes d'actionnement peuvent être agencés pour être actionnables à une seule main par l'utilisateur.

La languette d'actionnement peut être sollicitée élastiquement vers la position inactive.

La partie de distribution comprend une paroi s'étendant autour d'un axe de défilement depuis le corps, la paroi de la partie de distribution comportant deux portions d'actionnement en regard l'une de l'autre, la languette d'actionnement étant ménagée sur l'une des portions d'actionnement et au moins partiellement séparée d'une partie restante de ladite portion d'actionnement par une découpe. Ces dispositions permettent d'assurer une protection de la partie de bande déchirable comportant l'extrémité libre pour éviter qu'elle entre en contact avec des corps étrangers et des matières contaminantes.

La paroi de la partie de distribution peut présenter un bord d'extrémité opposé au corps, les portions d'actionnement s'étendant parallèlement l'une par rapport à l'autre et étant reliées l'une à l'autre par deux portions de liaison en regard l'une de l'autre, la découpe comportant un premier tronçon partant du bord d'extrémité à proximité de l'une des portions de liaison et s'éloignant du bord d'extrémité en direction du corps, et un deuxième tronçon parallèle au bord d'extrémité, la base de la languette d'actionnement s'étendant selon l'axe de défilement et étant reliée au corps par l'intermédiaire de l'autre portion de liaison.

Les organes d'actionnement peuvent comporter respectivement des languettes d'actionnement ménagées respectivement sur les portions d'actionnement, les découpes étant symétriques par rapport à l'axe de défilement.

Les portions d'actionnement peuvent être disposées de part et d'autre d'un plan de joint longitudinal médian comprenant l'axe de défilement, la paroi de la partie de distribution étant constituée par deux moitiés de paroi comprenant chacune l'une des languettes d'actionnement et au moins une partie de l'une des portions de liaison.

L'axe de défilement peut s'étendre dans un plan perpendiculaire à l'axe de pivotement et, notamment radialement par rapport à l'axe de pivotement.

Au moins l'un des bords de séparation peut présenter une arrête coupante en regard de l'autre bord de séparation.

Le bord de séparation en regard de l'arête coupante peut présenter une concavité adaptée pour recevoir l'arrête coupante.

La partie de distribution peut être venue de matière avec au moins une partie du corps.

Le corps peut comprendre en outre un boîtier délimitant un logement dans lequel s'étend le support et destiné à recevoir le rouleau de bande déchirable.

Le boîtier peut être constitué de deux moitiés de boîtier connectées l'une à l'autre selon un plan de joint transversal médian perpendiculaire à l'axe de pivotement.

Le distributeur peut comprendre en outre un couvercle adapté pour recouvrir la partie de distribution du dévidoir.

D'autres objets et avantages de l'invention apparaîtront à la lecture de la description qui suit d'un mode de réalisation particulier de l'invention donné à titre d'exemple non limitatif, la description étant faite en référence aux dessins annexés dans lesquels :

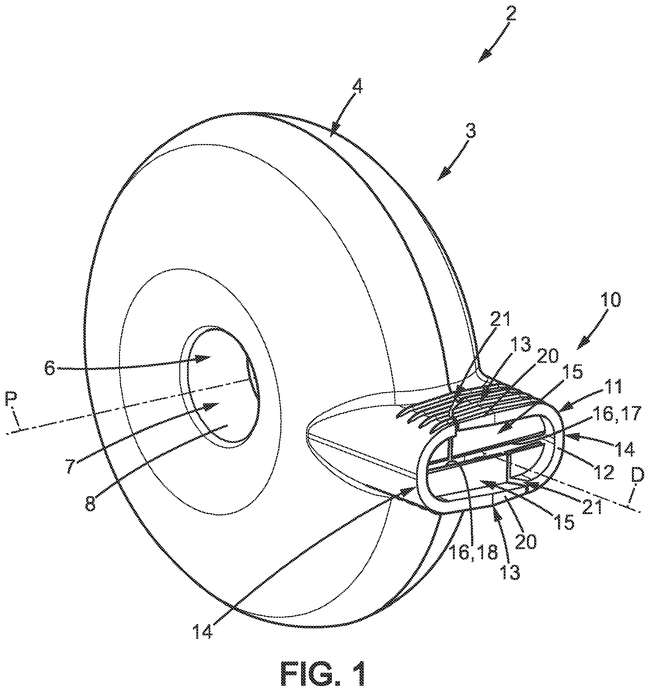

- la figure 1 est une représentation en perspective d'une première face latérale d'un dévidoir d'un distributeur de rouleau de bande déchirable selon un mode de réalisation de l'invention, le dévidoir comportant un corps adapté pour recevoir le rouleau de bande déchirable de manière pivotante autour d'un axe de pivotement, et une partie de distribution comprenant un dispositif de séparation adapté pour séparer du rouleau de bande déchirable un bout de bande déchirable, le dispositif de séparation comprenant les organes d'actionnement portant respectivement deux bords de séparation en regard l'un de l'autre, les organes d'actionnement étant rappelés élastiquement vers une position inactive et déplaçables par déformation élastique vers une position active,
- la figure 2 est une représentation en perspective d'une deuxième face latérale, opposée à la première face latérale, du dévidoir de la figure 1,
- la figure 3 est une représentation en vue de dessus du dévidoir de la figure 1,
- la figure 4 est une représentation en perspective d'une face intérieure d'une première coque formant avec une deuxième coque le dévidoir de la figure 1, la première coque comportant une première moitié de boîtier et une première moitié de support du corps du dévidoir, ainsi qu'une première moitié de paroi de la partie de distribution du dévidoir,
- la figure 5 est un agrandissement du détail référencé V sur la figure 4 de la partie de distribution de la première coque du dévidoir de la figure 1, illustrant l'un des bords de séparation de la partie de distribution,
- la figure 6 est une représentation en perspective d'une face intérieure de la deuxième coque du dévidoir de la figure 1, la deuxième coque comportant une deuxième moitié de boîtier et une deuxième moitié de support du corps du dévidoir, ainsi qu'une deuxième moitié de paroi de la partie de distribution du dévidoir,
- la figure 7 est une représentation en perspective du distributeur de bande déchirable comprenant en outre un couvercle recouvrant la partie de distribution du dévidoir.

Sur les figures, les mêmes références désignent des éléments identiques ou analogues.

Les figures 1 à 3 représentent un dévidoir 2 d'un distributeur 1 de rouleau de bande déchirable selon un mode de réalisation de l'invention.

Le rouleau de bande déchirable, non représenté, comporte une bande déchirable dont un bout de bande déchirable peut être séparé du reste du rouleau de bande déchirable par une action exercée à la main par un utilisateur. Dans le rouleau de bande déchirable, la bande déchirable est enroulée sur elle-même autour d'un axe central de telle manière que le rouleau de bande déchirable présente un trou selon l'axe central et la bande déchirable présente une extrémité libre. Le trou peut être délimité par un noyau cylindrique sur lequel la bande déchirable est enroulée ou directement par une première spire de bande déchirable au voisinage d'une extrémité opposée à l'extrémité libre. Selon une application particulière, la bande déchirable est une bande du type de celle décrite dans le document EP-A-2 058 424, utilisée dans le domaine médical ou dans le milieu sportif pour comprimer, immobiliser ou protéger une partie du corps d'une personne.

Le dévidoir 2, portable à la main par l'utilisateur, comprend un corps 3 dans lequel le rouleau de bande déchirable est monté de manière pivotante autour d'un axe de pivotement P. Le corps 3 comporte un boîtier 4 délimitant un logement 5 adapté pour recevoir le rouleau de bande déchirable, et un support 6 s'étendant selon l'axe de pivotement P à l'intérieur du boîtier 4 et adapté pour être placé dans le trou du rouleau de bande déchirable. Dans le mode de réalisation représenté, le boîtier 4 est globalement torique autour de l'axe de pivotement P et présente une ouverture centrale 7 délimitée par une paroi 8 cylindrique de section circulaire sur laquelle le support 6 est formé.

Le dévidoir 2 comprend également une partie de distribution 10 destinée à recevoir une partie de bande déchirable comportant l'extrémité libre. Dans le mode de réalisation représenté, la partie de distribution 10 est venue de matière avec le corps 3 et comprend une paroi 11 qui s'étend depuis le corps 3 jusqu'à un bord d'extrémité 12 opposé au corps 3, autour d'un axe de défilement D radial par rapport à l'axe de pivotement P. En variante, l'axe de défilement D pourrait avoir toute autre orientation appropriée et notamment toute autre orientation dans un plan perpendiculaire à l'axe de pivotement P.

La paroi 11 de la partie de distribution 10 est cylindrique de section oblongue avec :
- deux portions d'actionnement 13 en regard l'une de l'autre, parallèles l'une par rapport à l'autre, de part et d'autre d'un plan de joint longitudinal médian comprenant l'axe de défilement D et l'axe de pivotement P,
- deux portions de liaison 14, arrondies, en regard l'une de l'autre, reliant les portions d'actionnement 13 l'une à l'autre.

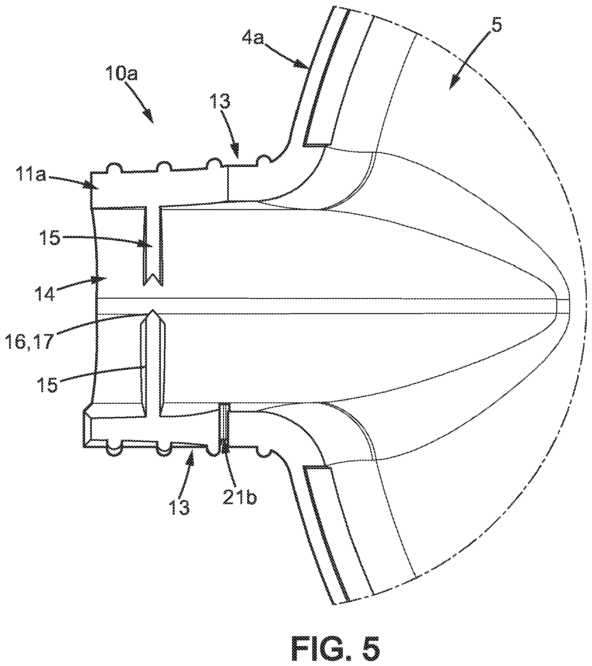

La partie de distribution 10 comprend également deux parois de séparation 15 s'étendant respectivement perpendiculairement depuis les portions d'actionnement 13, l'une vers l'autre, au voisinage de bord d'extrémité 12. Les parois de séparation 15 présentent respectivement des bords de séparation 16 opposés aux portions d'actionnement 13 respectives et disposés en regard l'un de l'autre. Dans le mode de réalisation représenté, comme particulièrement visible sur la figure 5, l'un des bords de séparation 16 présente une arrête coupante 17 et l'autre bord de séparation 16 présente une concavité 18 adaptée pour recevoir l'arrête coupante 17.

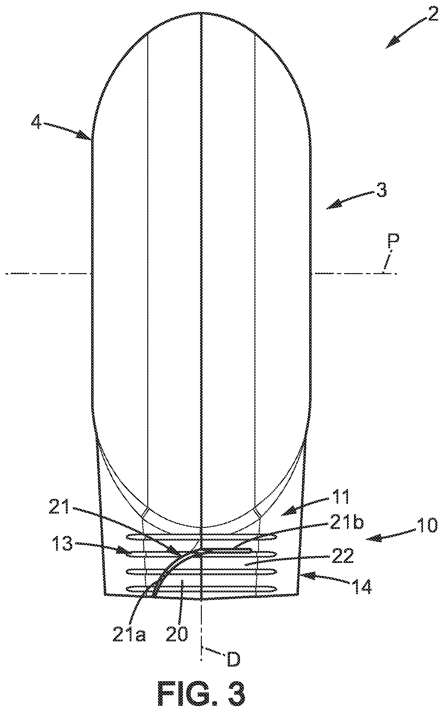

Une languette d'actionnement 20 est ménagée sur chacune des portions d'actionnement 13. En particulier, chaque languette d'actionnement 20 est séparée d'une partie restante de la portion d'actionnement 13 par une découpe 21. Dans le mode de réalisation représenté, comme particulièrement visible sur la figure 3, la découpe 21 s'étendant sur une partie de la portion d'actionnement 13 et comporte :
- un premier tronçon 21a, arrondi, partant du bord d'extrémité 12 à proximité de l'une des portions de liaison 14 et s'éloignant du bord d'extrémité 12 en direction du corps 3, le premier tronçon 21a se prolongeant sur la paroi de séparation 15 attenante à la portion d'actionnement 13, et
- un deuxième tronçon 21b parallèle au bord d'extrémité 12.

Chaque languette d'actionnement 20 comprend alors une base 22 qui s'étend selon l'axe de défilement D et qui est reliée au corps 3 du dévidoir 2 par l'intermédiaire de la portion de liaison 14 opposée à celle depuis laquelle s'étend le premier tronçon 21a de la découpe 21.

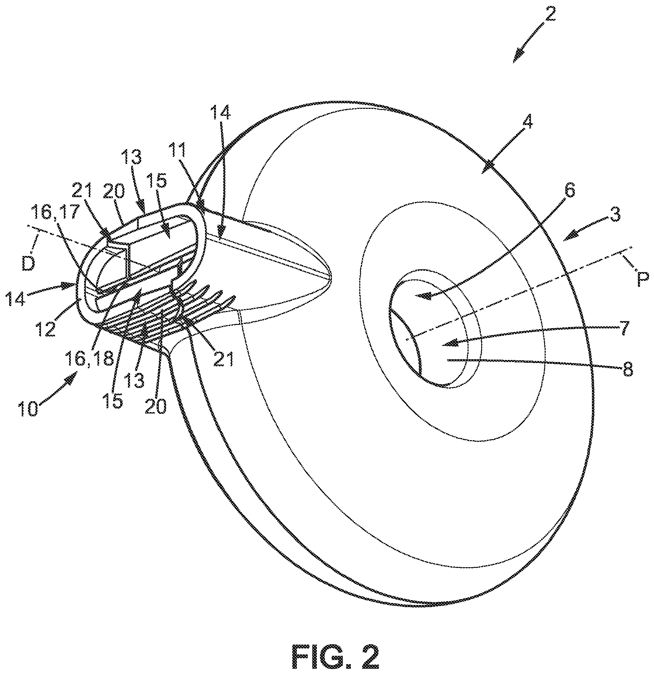

Comme il ressort des figures 1 et 2, les découpes 21 sur les portions d'actionnement 13 sont symétriques par rapport à l'axe de défilement D.

Chaque languette d'actionnement 20 est sollicitée élastiquement vers une position inactive dans laquelle les bords de séparation 16 sont écartés l'un de l'autre de manière à pouvoir faire défiler la bande déchirable entre eux. Chaque languette d'actionnement 20 est, par ailleurs, déplaçable par déformation élastique au niveau de sa base 22 vers une position active dans laquelle les bords de séparation 16 sont rapprochés l'un de l'autre de manière à pouvoir venir en contact avec la bande déchirable. Dans la position active, les bords de séparation 16 peuvent réaliser une coupe nette du bout de bande déchirable. Les languettes d'actionnement 20 forment alors des organes d'actionnement d'un dispositif de séparation actionnables à la main, voire à une seule main, par l'utilisateur.

L'invention n'est pas limitée à un dispositif de séparation comprenant des organes d'actionnement sous la forme de languettes d'actionnement 20 tel que décrit précédemment. En variante, tout dispositif de séparation écarté du support 6 et comprenant des organes d'actionnement déplaçables l'un par rapport à l'autre entre une position inactive et une position active permettant de séparer du rouleau de bande déchirable un bout de bande déchirable pourrait être prévu. En particulier, la languette d'actionnement 20 pourrait être reliée au corps 3 par une base 22 s'étendant perpendiculairement à l'axe de défilement D, une découpe 21 en deux tronçons distincts, de part et d'autre de la languette d'actionnement 20, séparant entièrement la languette d'actionnement 20 d'une partie restante de la portion d'actionnement 13. Par ailleurs, seul l'un des organes d'actionnement pourrait être réalisé sous la forme d'une languette d'actionnement 20 déformable élastiquement. En outre, les bords de séparation 16 pourraient être conformés de toute autre manière appropriée et notamment pour assurer un simple un maintien de la bande déchirable.

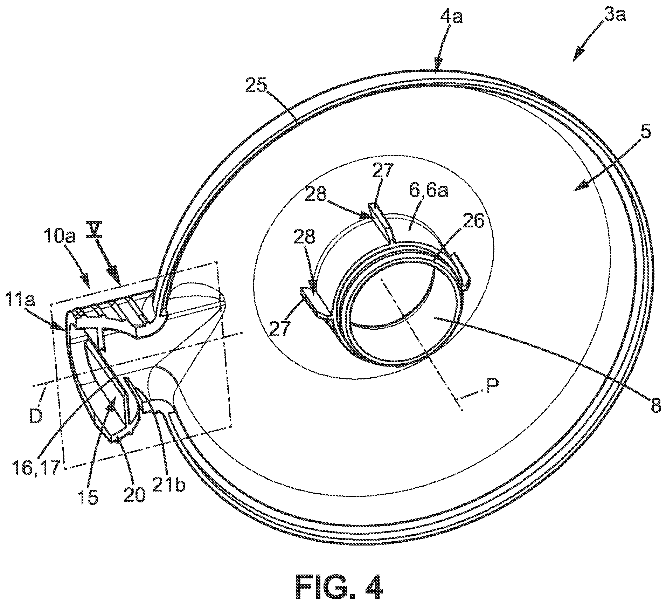

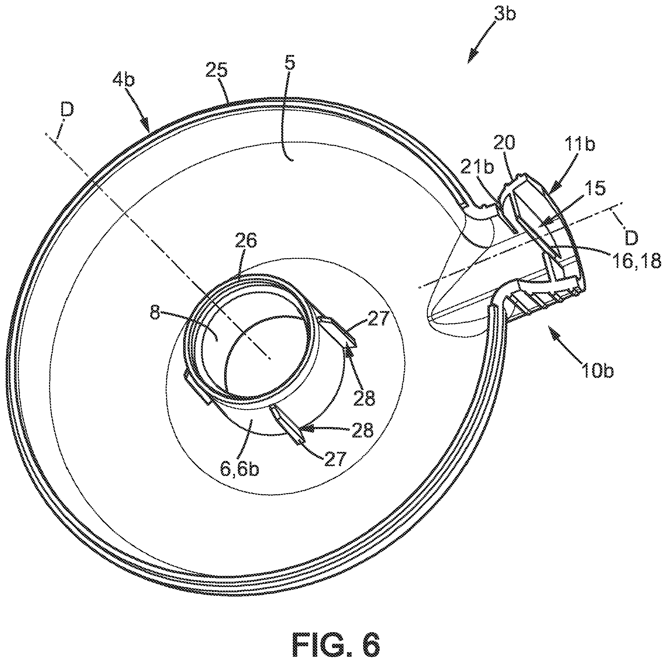

Comme représenté sur les figures 4 et 6, le dévidoir 2 peut être formé par l'assemblage d'une première demi-coque 3a, 10a et d'une deuxième demi-coque 3b, 10b, toutes deux réalisées par exemple par moulage d'une matière plastique. La première coque 3a, 10a comporte une première moitié de boîtier 4a et une première moitié de support 6a du corps 3 du dévidoir 2, ainsi qu'une première moitié de paroi 11a de la partie de distribution 10 du dévidoir 2. De la même manière, la deuxième coque 3b, 10b comporte une deuxième moitié de boîtier 4b et une deuxième moitié de support 6b du corps 3 du dévidoir 2, ainsi qu'une deuxième moitié de paroi 11b de la partie de distribution 10 du dévidoir 2.

Les première 4a et deuxième 4b moitiés de boîtier peuvent être connectées l'une à l'autre selon un plan de joint transversal médian, perpendiculaire à l'axe de pivotement P. La connexion première 4a et deuxième 4b moitiés de boîtier peut notamment être réalisée par emmanchement d'une bordure périphérique 25 de l'une des moitiés de boîtier 4a, 4b dans une bordure périphérique 25 de l'autre moitié de boîtier 4a, 4b.

Les première 6a et deuxième 6b moitiés de support peuvent être connectées l'une à l'autre sensiblement selon le même plan de joint transversal médian que celui du boîtier 4. A nouveau, la connexion première 6a et deuxième 6b moitiés de support peut notamment être réalisée par emmanchement d'une bordure périphérique 26 de l'une des moitiés de support 6a, 6b dans une bordure périphérique 26 de l'autre moitié de support 6a, 6b. Comme représenté sur les figures 4 et 6, le support 6 peut comporter une surface support constituée par des surfaces élémentaires 27 discrètes portées par des saillies 28 équiréparties s'étendant radialement par rapport à l'axe de pivotement P depuis une surface intérieure de la paroi 8 délimitant l'ouverture centrale 7 du boîtier 4. En variante, la surface support pourrait être réalisée de toute autre manière appropriée.

Les première 11a et deuxième 11b moitiés de paroi de la partie de distribution 10 comprennent chacune l'une des languettes d'actionnement 20 avec une partie de la paroi de séparation 15 attenante et le deuxième tronçon 21b de la découpe 21, la paroi de liaison 14 à laquelle la base 22 de la languette d'actionnement 20 est reliée et la partie restante de la portion d'actionnement 13 sur laquelle l'autre languette d'actionnement 20 est ménagée.

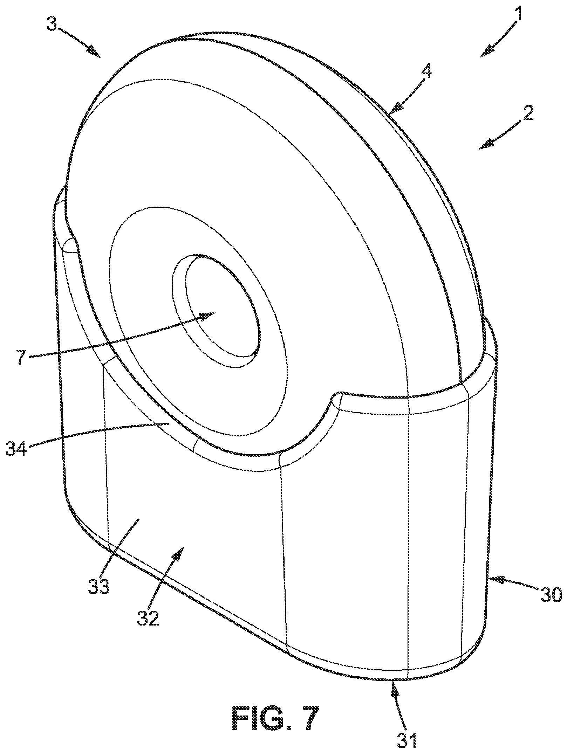

Sur la figure 7, le distributeur 1 comprend en outre un couvercle 30 adapté pour recouvrir la partie de distribution 10 du dévidoir 2. En particulier, le couvercle 30 comporte un fond 31 généralement plat sur lequel le distributeur 1 peut reposer, et une surface latérale 32 qui s'étend perpendiculairement depuis le fond 31 de manière à délimiter un espace adapté pour recevoir la partie de distribution 10 du dévidoir 2 et une partie du corps 3, par exemple une moitié du corps 3. Sur des portions 33 parallèles de la paroi latérale 32 du couvercle 30 destinées à s'étendre perpendiculairement à l'axe de pivotement P, des échancrures 34 peuvent être prévues pour laisser l'ouverture centrale 7 du boîtier 4 accessible.

## Revendications

1. Distributeur (1) pour rouleau de bande déchirable, le rouleau de bande déchirable comportant une bande déchirable enroulée sur elle-même autour d'un axe central de telle manière que le rouleau de bande déchirable présente un trou selon l'axe central et que la bande déchirable présente une extrémité libre, le distributeur (1) comprenant un dévidoir (2) comportant :
- un corps (3) comprenant un support (6) s'étendant selon un axe de pivotement (P) et destiné à être placé dans le trou du rouleau de bande déchirable de manière à permettre un pivotement du rouleau de bande déchirable selon l'axe de pivotement (P),
- une partie de distribution (10) destinée à recevoir une partie de bande déchirable comportant l'extrémité libre, la partie de distribution (10) comprenant un dispositif de séparation écarté du support (6) et adapté pour séparer du rouleau de bande déchirable un bout de bande déchirable, la partie de distribution (10) comprenant une paroi (11) qui s'étend autour d'un axe de défilement (D) depuis le corps (3), la paroi (11) de la partie de distribution (10) comportant deux portions d'actionnement (13) en regard l'une de l'autre,
dans lequel le dispositif de séparation comprend deux organes d'actionnement (20) actionnables par un utilisateur, les organes d'actionnement (20) portant respectivement deux bords de séparation (16) en regard l'un de l'autre, les organes d'actionnement (20) étant déplaçables l'un par rapport à l'autre entre une position inactive dans laquelle les bords de séparation (16) sont écartés l'un de l'autre de manière à pouvoir faire défiler la bande déchirable entre lesdits bords de séparation (16), et une position active dans laquelle les bords de séparation (16) sont rapprochés l'un de l'autre de manière à pouvoir venir en contact avec la bande déchirable,
le distributeur étant **caractérisé en ce que** au moins l'un des organes d'actionnement est une languette d'actionnement (20) ménagée sur l'une des portions d'actionnement (13), la languette d'actionnement (20) étant partiellement séparée d'une partie restante de ladite portion d'actionnement (13) par une découpe (21) et s'étendant depuis une base (22) reliée à ladite partie restante, la languette d'actionnement (20) étant déplaçable entre les positions active et inactive par déformation élastique au niveau de la base (22).

2. Distributeur (1) selon la revendication 1, dans lequel la languette d'actionnement (20) est sollicitée élastiquement vers la position inactive.

3. Distributeur (1) selon l'une quelconque des revendications 1 et 2, dans lequel la paroi (11) de la partie de distribution (10) présente un bord d'extrémité (12) opposé au corps (3), les portions d'actionnement (13) s'étendant parallèlement l'une par rapport à l'autre et étant reliées l'une à l'autre par deux portions de liaison (14) en regard l'une de l'autre, la découpe (21) comportant un premier tronçon (21a) partant du bord d'extrémité (12) à proximité de l'une des portions de liaison (14) et s'éloignant du bord d'extrémité (12) en direction du corps (3), et un deuxième tronçon (21b) parallèle au bord d'extrémité (12), la base (22) de la languette d'actionnement (20) s'étendant selon l'axe de défilement (D) et étant reliée au corps (3) par l'intermédiaire de l'autre portion de liaison (14).

4. Distributeur (1) selon l'une quelconque des revendications 1 à 3, dans lequel les organes d'actionnement comportent respectivement des languettes d'actionnement (20) ménagées respectivement sur les portions d'actionnement (13), les découpes (21) étant symétriques par rapport à l'axe de défilement (D).

5. Distributeur (1) selon l'une quelconque des revendications 1 à 4, dans lequel les portions d'actionnement (13) sont disposées de part et d'autre d'un plan de joint longitudinal médian comprenant l'axe de défilement (D), la paroi (11) de la partie de distribution (10) étant constituée par deux moitiés de paroi (11a, 11b) comprenant chacune l'une des languettes d'actionnement (20) et au moins une partie de l'une des portions de liaison (14).

6. Distributeur (1) selon l'une quelconque des revendications 1 à 5, dans lequel l'axe de défilement (D) s'étend dans un plan perpendiculaire à l'axe de pivotement (P) et, notamment radialement par rapport à l'axe de pivotement (P).

7. Distributeur (1) selon l'une quelconque des revendications 1 à 6, dans lequel au moins l'un des bords de séparation (16) présente une arrête coupante (17) en regard de l'autre bord de séparation (16).

8. Distributeur (1) selon la revendication 7, dans lequel le bord de séparation (16) en regard de l'arête coupante (17) présente une concavité (18) adaptée pour recevoir l'arrête coupante (17).

9. Distributeur (1) selon l'une quelconque des revendications 1 à 8, dans lequel la partie de distribution (10) est venue de matière avec au moins une partie du corps (3).

10. Distributeur (1) selon l'une quelconque des revendications 1 à 9, dans lequel le corps (3) comprend en outre un boîtier (4) délimitant un logement (5) dans lequel s'étend le support (6) et destiné à recevoir le rouleau de bande déchirable.

11. Distributeur (1) selon la revendication 10, dans lequel le boîtier (4) est constitué de deux moitiés de boîtier (4a, 4b) connectées l'une à l'autre selon un plan de joint transversal médian perpendiculaire à l'axe de pivotement (P).

12. Distributeur (1) selon l'une quelconque des revendications 1 à 11, comprenant en outre un couvercle (30) adapté pour recouvrir la partie de distribution (10) du dévidoir (2).

## Patentansprüche

1. Spender (1) für eine Rolle reißbares Band, wobei die Rolle reißbares Band ein reißbares Band umfasst, das auf sich selbst um eine Mittelachse so aufgewickelt ist, dass die Rolle des reißbaren Bandes ein Loch entlang der Mittelachse aufweist und dass das reißbare Band ein freies Ende aufweist, wobei der Spender (1) eine Abrollvorrichtung (2) mit:
- einen Körper (3) mit einer Halterung (6), die sich entlang einer Drehachse (P) erstreckt aufweist, und dazu bestimmt ist, in das Loch der abreißbaren Bandrolle eingesetzt zu werden, um einen Schwenk der abreißbaren Bandrolle entlang der Drehachse (P) zu ermöglichen,
- einen Abgabebereich (10) zur Aufnahme eines abreißbaren Streifenabschnitts mit dem freien Ende, wobei der Abgabebereich (10) eine von der Halterung (6) beabstandete Trennvorrichtung aufweist und geeignet ist, einen abreißbaren Streifenabschnitt von der abreißbaren Streifenrolle zu trennen, wobei der Abgabebereich (10) eine Wand (11) aufweist, die erstreckt sich um eine Scrollachse (D) vom Körper (3) aus, wobei die Wand (11) des Verteilerteils (10) zwei einander zugewandte Betätigungsabschnitte (13) aufweist,
wobei die Trennvorrichtung zwei Stellglieder (20) umfasst, die von einem Benutzer bedient werden können, wobei die Stellglieder (20) jeweils zwei Trennkanten (16) tragen, die einander zugewandt sind, wobei die Stellglieder (20), die relativ zueinander zwischen einer inaktiven Position, in der die Trennkanten (16) voneinander beabstandet sind, um das abreißbare Band zwischen den Trennkanten (16) scrollen zu können, und einer aktiven Position, in der die Trennkanten (16) näher beieinander bewegt werden, um mit dem abreißbaren Band in Kontakt kommen zu können,
wobei der Verteiler **dadurch gekennzeichnet ist, dass** mindestens einer der Stellglieder eine Betätigungslasche (20) ist, die an einem der Betätigungsabschnitte (13) vorgesehen ist, wobei die Betätigungslasche (20) teilweise von einem verbleibenden Teil des Betätigungsabschnitts (13) durch einen Ausschnitt (21) getrennt ist und sich von einer mit dem verbleibenden Teil verbundenen Basis (22) erstreckt, wobei die Betätigungslasche (20) zwischen den Positionen aktiv und inaktiv durch elastische Verformung an der Basis (22) beweglich ist.

2. Spender (1) nach Anspruch 1, wobei die Betätigungslasche (20) elastisch in die inaktive Position belastet wird.

3. Spender (1) nach einem der Ansprüche 1 und 2, wobei die Wand (11) des Spendeteils (10) eine Endkante (12) gegenüber dem Körper (3), wobei sich die Betätigungsabschnitte (13) parallel zueinander erstrecken und durch zwei einander zugewandte Verbindungsabschnitte (14) miteinander verbunden sind, wobei der Ausschnitt (21) einen ersten Abschnitt (21a) aufweist, der von der Endkante (12) nahe einem der Verbindungsabschnitte (14) ausgeht und von der Endkante (12) zum Körper (3) zeigt, und einen zweiten Abschnitt (21b) parallel zur Endkante (12), wobei sich die Basis (22) der Betätigungslasche (20) entlang der Laufachse (D) erstreckt und über den anderen Verbindungsabschnitt (14) mit dem Körper (3) verbunden ist.

4. Spender (1) nach einem der Ansprüche 1 bis 3, bei dem die Stellglieder jeweils Betätigungszungen (20) aufweisen, die auf den Betätigungsabschnitten (13) vorgesehen sind, wobei die Ausschnitte (21) symmetrisch zur Laufachse (D) sind.

5. Spender (1) nach einem der Ansprüche 1 bis 4, wobei die Betätigungsabschnitte (13) auf beiden Seiten einer mittleren Längsteilungslinie angeordnet sind, die die Laufachse (D), die Wand (11) des Teils von Verteilung (10), die aus zwei Wandhälften (11a, 11b) besteht, die jeweils eine der Betätigungszungen (20) und mindestens einen Teil eines der Verbindungsabschnitte (14) umfassen.

6. Spender (1) nach einem der Ansprüche 1 bis 5, wobei sich die Scrollachse (D) in einer Ebene senkrecht zur Drehachse (P) und insbesondere radial zur Drehachse (P) erstreckt.

7. Spender (1) nach einem der Ansprüche 1 bis 6, wobei mindestens eine der Trennkanten (16) eine Schneide (17) gegenüber der anderen Trennkante (16) aufweist.

8. Spender (1) nach Anspruch 7, wobei die Trennkante (16) gegenüber der Schneidkante (17) eine Konkavität (18) aufweist, die zur Aufnahme der Schneidkante (17) ausgebildet ist.

9. Spender (1) nach einem der Ansprüche 1 bis 8, wobei der Spendeabschnitt (10) aus einem Material mit mindestens einem Teil des Körpers (3) stammt.

10. Spender (1) nach einem der Ansprüche 1 bis 9, bei dem die Körper (3), die ferner ein Gehäuse (4) umfassen, das ein Gehäuse (5) begrenzt, in dem sich die Halterung (6) erstreckt und dazu bestimmt ist, die Rolle des reißbaren Bandes aufzunehmen.

11. Spender (1) nach Anspruch 10, wobei das Gehäuse (4) aus zwei Gehäusehälften (4a, 4b) besteht, die in einer zentralen Querverbindungsebene senkrecht zur Drehachse (P) miteinander verbunden sind.

12. Spender (1) nach einem der Ansprüche 1 bis 11, ferner umfassend eine Abdeckung (30), die geeignet ist, den Spendeabschnitt (10) der Abrollvorrichtung (2) abzudecken.

## Claims

1. Dispenser (1) for a roll of tear-off strip, the roll of tear-off strip including a tear-off strip wound onto itself about a central axis such that the roll of tear-off strip has a hole along the central axis and the tear-off strip has a free end, the dispenser (1) comprising a reel (2) including:
- a body (3) comprising a support (6) extending along a pivoting axis (P) and intended to be placed in the hole of the roll of tear-off strip so as to enable pivoting of the roll of tear-off strip along the pivoting axis (P),
- a dispensing part (10) intended to a receive a portion of tear-off strip including the free end, the dispensing part (10) comprising a separating device removed from the support (6) and suitable for separating from the roll of tear-off strip a piece of tear-off strip, the dispensing part (10) comprising a wall (11) extending about a travel axis (D) from the body (3), the wall (11) of the dispensing part (10) including two actuating portions (13) facing one another,
wherein the separating device comprises two actuating members (20) which can be actuated by a user, the actuating members (20) supporting respectively two separating edges (16) facing one another, the actuating members (20) being movable relative to one another between an idle position wherein the separating edges (16) are spaced apart from one another so as to enable the tear-off strip to travel between said separating edges (16), and an active position wherein the separating edges (16) are close to one another so as to be able to come into contact with the tear-off strip,
the dispenser being **characterised in that** at least one of the actuating members is an actuating tab (20) provided on one of the actuating portions (13), the actuating tab (20) being partially separated from a remaining portion of said actuating portion (13) by a cut area (21) and extending from a base (22) connected to said remaining portion, the actuating tab (20) being movable between the active and idle positions by elastic deformation at the level of the base (22).

2. Dispenser (1) according to claim 1, wherein the actuating tab (20) is stressed elastically to the idle position.

3. Dispenser (1) according to any one of claims 1 and 2, wherein the wall (11) of the dispensing part (10) has an end edge (12) opposite the body (3), the actuating portions (13) extending parallel in relation to one another and being connected to one another by two connecting portions (14) facing one another, the cut area (21) including a first segment (21a) starting from the end edge (12) in the vicinity of one of the connecting portions (14) and moving away from the end edge (12) towards the body (3), and a second segment (21b) parallel with the end edge (12), the base (22) of the actuating tab (20) extending along the travel axis (D) and being connected to the body (3) by means of the other connecting portion (14).

4. Dispenser (1) according to any one of claims 1 to 3, wherein the actuating members include respectively actuating tabs (20) provided respectively on the actuating portions (13), the cut areas (21) being symmetrical relative to the travel axis (D).

5. Dispenser (1) according to any one of claims 1 to 4, wherein the actuating portions (13) are disposed on either side of a median longitudinal joint plane comprising the travel axis (D), the wall (11) of the dispensing part (10) consisting of two half-walls (11a, 11b) each comprising one of the actuating tabs (20) and at least a part of one of the connecting portions (14).

6. Dispenser (1) according to any one of claims 1 to 5, wherein the travel axis (D) extends in a perpendicular plane to the pivoting axis (P) and, particularly radially relative to the pivoting axis (P),

7. Dispenser (1) according to any one of claims 1 to 6, wherein at least one of the separating edges (16) has a cutting edge (17) facing the other separating edge (16).

8. Dispenser (1) according to claim 7, wherein the separating edge (16) facing the cutting edge (17) has a concavity (18) suitable for receiving the cutting edge (17).

9. Dispenser (1) according to any one of claims 1 to 8, wherein the dispensing part (10) is integrally formed with at least one part of the body (3).

10. Dispenser (1) according to any one of claims 1 to 9, wherein the body (3) further comprises a casing (4) defining a housing (5) wherein extends the support (6) and intended to receive the roll of tear-off strip.

11. Dispenser (1) according to claim 10, wherein the casing (4) consists of two half-casings (4a, 4b) connected to one another along a median transverse joint plane perpendicular to the pivoting axis (P).

12. Dispenser (1) according to any one of claims 1 to 11, further comprising a cover (30) suitable for covering the dispensing part (10) of the reel (2).
